(19) Europäisches Patentamt
European Patent Office
Office européen des brevets

(11) EP 1 374 835 B1

(12) FASCICULE DE BREVET EUROPEEN

(45) Date de publication et mention de la délivrance du brevet: **24.08.2005 Bulletin 2005/34**

(51) Int Cl.[7]: **A61K 7/02**

(21) Numéro de dépôt: **03011823.6**

(22) Date de dépôt: **26.05.2003**

# (54) Fond de teint émulsion eau-dans-huile

Öl-in-Wasser Emulsion als Grundierungspräparat

Foundation composition which is an oil-in-water emulsion

(84) Etats contractants désignés:
**AT BE BG CH CY CZ DE DK EE ES FI FR GB GR HU IE IT LI LU MC NL PT RO SE SI SK TR**

(30) Priorité: **26.06.2002 FR 0207937**
**02.10.2002 FR 0212190**

(43) Date de publication de la demande:
**02.01.2004 Bulletin 2004/01**

(73) Titulaire: **L'OREAL**
**75008 Paris (FR)**

(72) Inventeurs:
- **Gardel, Nadia**
  **92340 Bourg la Reine (FR)**
- **Barrois, Veronique**
  **75011 Paris (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 0 595 683**
**EP-A- 0 856 309**
**EP-A- 1 055 423**
**WO-A-99/30681**
**FR-A- 2 776 513**
**FR-A- 2 776 514**
**FR-A- 2 776 515**
**FR-A- 2 789 313**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 374 835 B1

## Description

La présente invention a pour objet une composition cosmétique de fond de teint sous forme d'émulsion eau-dans huile comprenant des tensioactifs siliconés et une huile volatile . L'invention a également pour objet un procédé de maquillage de la peau comprenant l'application du fond de teint sur la peau.

La composition de fond de teint est une composition de maquillage de la peau d'être humain. La composition selon l'invention peut être un fond de teint à appliquer sur le visage ou le cou, un produit anti-cernes, une crème teintée, un composition de maquillage du corps.

Les compositions de fond de teint sont couramment employées pour apporter une couleur esthétique à la peau, notamment au visage, mais également pour camoufler les imperfections de la peau telles que les rougeurs, les taches.

Il est connu du document FR-A-2686510 des émulsions de fond de teint eau-dans-huile comprenant comme tensioactif un alkyl diméthicone copolyol, en particulier un cétyl diméthicone copolyol. Or on a constaté que lorsque ces émulsions contiennent une quantité importante (plus de 15 % en poids) d'huiles volatiles, comme par exemple la cyclopentasiloxane, l'émulsion fluide n'est pas stable au cours du temps : l'émulsion après un stockage de 2 mois, voire de 4 mois, à température ambiante (25 °C),relargue de l'huile à la surface de la composition et n'est donc plus homogène. L'utilisatrice doit alors bien agiter la composition avant son utilisation. Si la composition n'est pas agitée ou bien mal agitée, l'application de cette composition sur la peau laisse une sensation de gras inconfortable et le maquillage obtenu n'est pas homogène, des traces de couleur sont visibles sur la peau.

La demande WO99/30681 décrit une composition de maquillage sous forme d'émulsion eau-dans-silicone comprenant un hydroxyacide, un agent filtrant les U.V, un agent hydratant, un antioxydant, un pigment, un émulsionnant, un composé siliconé et de l'eau. La composition appliquée sur la peau protège la peau du rayonnement solaire et réduit l'apparition des rides.
Le composé siliconé est choisi de préférence parmi cyclométhicone, diméthicone et dérivés, cyclométhicone polyol, diméthicone polyol, cétyl diméthicone copolyol et leurs mélanges. Des émulsionnants siliconés sont choisis parmi diméthicone polyol ou céthyldiméthicone copolyol.

La demande FR2776515 décrit une émulsion cosmétique eau-dans-huile contenant un diméthicone copolyol et un tensioactif de type ester de glycol. La phase grasse de l'émulsion comprend une huile de silicone, volatile ou non et peut comprendre une huile hydrocarbonée en une teneur allant jusqu'à 40 % en poids par rapport au poids total de la phase grasse. L'émulsion est stable et ne transfère pas après son application sur la peau.

La demande FR2789313 décrit une composition cosmétique sous forme d'émulsion eau-dans-huile comprenant un émulsionnant siliconé, une huile hydrocarbonée à chaîne ramifiée et une huile de silicone volatile, la quantité totale d'huile hydrocarbonée à chaîne ramifiée et d'huile de silicone volatile représentant au moins 50 % en poids de la phase huileuse. Cette composition est fluide, a un toucher frais, agréable à l'application, facile à étaler, présente des propriétés émollientes, laisse la peau douce, mate et non collante. L'émulsionnant siliconé est un diméthicone copolyol ou un alkyl diméthicone copolyol.

La demande EP595683 décrit une émulsion cosmétique eau-dans-huile comprenant un tensioactif siliconé et un fluorohydrocarbure. Le tensioactif siliconé est un diméthicone copolyol ou un alkyldiméthicone copolyol. L'émulsion est stable.

Le but de la présente invention est de disposer d'une composition de fond de teint ayant une bonne stabilité après un stockage à température ambiante (25 °C) pendant au moins 2 mois, voire 4 mois, et permettant d'obtenir un maquillage homogène de la peau.

Les inventeurs ont découvert qu'un tel fond de teint pouvait être obtenu en utilisant un alkyl diméthicone copolyol, un diméthicone copolyol et un mélange d'huiles volatiles en forte teneur.

De façon plus précise, l'invention a pour objet un fond de teint sous forme d'émulsion eau-dans-huile comprenant une phase grasse, une phase aqueuse, un alkyl $C_8$-$C_{22}$ diméthicone copolyol, un diméthicone copolyol présent en une teneur allant de 5 % à 10 % en poids, par rapport au poids total de l'émulsion, des pigments enrobés hydrophobes, la phase grasse comprenant au moins 30 % en poids, par rapport au poids total de l'émulsion, de phase grasse volatile comprenant :

- au moins 6 % en poids, par rapport au poids total de l'émulsion, d' au moins une huile volatile hydrocarbonée, et
- au moins une huile volatile choisie parmi les huiles volatiles siliconées, les huiles volatiles fluorées, et leurs mélanges.

L'invention a aussi pour objet un procédé cosmétique de maquillage non thérapeutique de la peau comprenant l'application sur la peau d'une composition telle que définie précédemment.

L'invention a encore pour objet l'utilisation d'une composition telle que définie précédemment pour obtenir un maquillage homogène de la peau.

L'invention a également pour objet l'utilisation d'un alkyl $C_8$-$C_{22}$ diméthicone copolyol et d'un diméthicone copolyol dans une composition de fond de teint sous forme d'émulsion eau-dans-huile contenant une phase grasse, une phase aqueuse, des pigments enrobés hydrophobes, la phase grasse comprenant au moins 30 % en poids, par rapport au poids total de l'émulsion, de phase grasse volatile comprenant :

- au moins 6 % en poids, par rapport au poids total de l'émulsion, d' au moins une huile volatile hydrocarbonée, et
- au moins une huile volatile choisie parmi les huiles volatiles siliconées, les huiles volatiles fluorées, et leurs mélanges,

le diméthicone copolyol étant présent en une teneur allant de 5 % à 10 % en poids, par rapport au poids total de l'émulsion,
pour obtenir une émulsion stable et/ou homogène et/ou pour obtenir un maquillage homogène de la peau.

L'émulsion selon l'invention présente une très bonne stabilité à température ambiante (25 °C), notamment après un stockage de 2 mois ou mieux de 4 mois. Le fond de teint s'applique facilement sur la peau, avec une sensation d'onctuosité, de douceur et non grasse, se répartit de façon homogène sur la peau et sèche rapidement après l'application. Le maquillage obtenu est homogène, sans laisser de trace sur la peau et présente une bonne tenue dans le temps de la matité.

L'alkyl $C_8$-$C_{22}$ diméthicone copolyol présent dans le fond de teint selon l'invention est un poly méthyl alkyl ($C_8$-$C_{22}$) diméthyl méthyl siloxane oxypropyléné et/ou oxyéthyléné.

L'alkyl $C_8$-$C_{22}$ diméthicone copolyol est avantageusement un composé de formule (I) suivante :

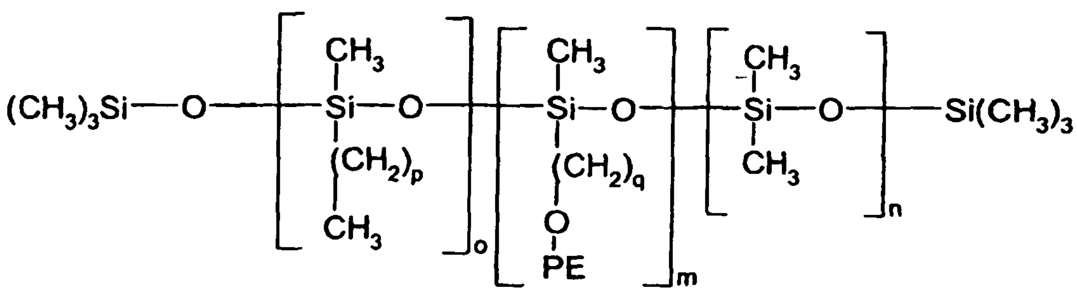

(I)

dans laquelle :

- PE représente $(-C_2H_4O)_x$-$(C_3H_6O)_y$-R, R étant choisi parmi un atome d'hydrogène et un radical alkyle de 1 à 4 atomes de carbone, x allant de 0 à 100 et y allant de 0 à 80, x et y n'étant pas simultanément 0
- m allant de 1 à 40
- n allant de 10 à 200
- o allant de 1 à 100
- p allant de 7 et 21
- q allant de 0 à 4

et de préférence :

R=H
m = 1 à 10
n = 10 à 100
o = 1 à 30
p = 15
q = 3

Comme alkyl $C_8$-$C_{22}$ diméthicone copolyol, on peut citer le cétyl diméthicone copolyol comme le produit commercialisé sous la dénomination Abil® EM-90 par la société Goldschmidt.

L'alkyl $C_8$-$C_{22}$ diméthicone copolyol peut être présent dans l'émulsion selon l'invention en une teneur allant de 0,5 % à 2 % en poids, par rapport au poids total de l'émulsion, notamment allant de 0,6 % à 2 % en poids, mieux allant de 0,7 % à 2 % en poids, voire allant de 0,8 % à 2 % en poids, et de préférence allant de 0,5 % à 1,5 % en poids, notamment allant de 0,6 % à 1,5 % en poids, mieux allant de 0,7 % à 1,5 % en poids,voire allant de 0,8 % à 1,5 % en

poids.

Le diméthicone copolyol présent dans le fond de teint selon l'invention est un polydiméthyl méthyl siloxane oxypropyléné et/ou oxyéthyléné.

On peut utiliser comme diméthicone copolyol ceux répondant à la formule (II) suivante :

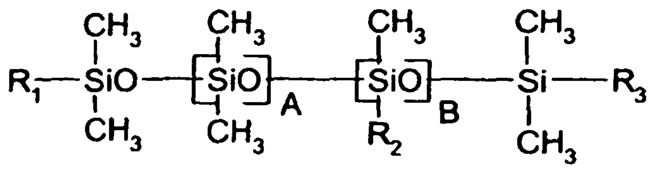

(II)

dans laquelle :

$R_1$, $R_2$, $R_3$, indépendamment les uns des autres, représentent un radical alkyle en $C_1$-$C_6$ ou un radical $-(CH_2)_x$ - $(OCH_2CH_2)_y$ - $(OCH_2CH_2CH_2)_z$ - $OR_4$, au moins un radical $R_1$, $R_2$ ou $R_3$ n'étant pas un radical alkyle ; $R_4$ étant un hydrogène, un radical alkyle en $C_1$-$C_3$ ou un radical acyle en $C_2$-$C_4$ ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (II), $R_1 = R_3$ = radical méthyle, x est un nombre entier allant de 2 à 6 et y est un nombre entier allant de 4 à 30. $R_4$ est en particulier un hydrogène.

On peut citer, à titre d'exemple composés de formule (II), les composés de formule (III) :

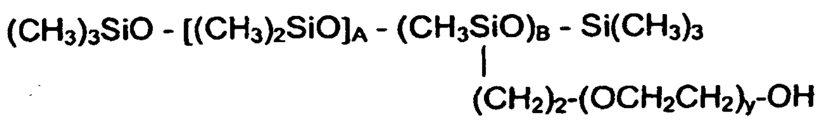

(III)

dans laquelle A est un nombre entier allant de 20 à 105, B est un nombre entier allant de 2 à 10 et y est un nombre entier allant de 10 à 20.

On peut également citer à titre d'exemple de composés siliconés de formule (II), les composés de formule (IV):

$$HO - (CH_2CH_2O)_y-(CH_2)_3 - [(CH_3)_2SiO]_{A'} - [(CH_3)_2Si] - (CH_2)_3 - (OCH_2CH_2)_y - OH \quad (IV)$$

dans laquelle A' et y sont des nombres entiers allant de 10 à 20.

On peut utiliser comme diméthicone copolyol ceux vendus sous les dénominations DC 5329®, DC 7439-146®, DC 2-5695®, Q4-3667® par la société Dow Coming ; Kif-6013®, KF-6015®, KF-6016®, KF-6017® par la société Shin-Etsu.

Les composés DC 5329®, DC 7439-146®, DC 2-5695® sont des composés de formule (III) où respectivement A est 22, B est 2 et y est 12 ; A est 103, B est 10 et y est 12 ; A est 27, B est 3 et y est 12.

Le diméthicone copolyol peut être de préférence présent dans l'émulsion selon l'invention en une teneur allant de 5 % à 8 % en poids, par rapport au poids total de l'émulsion, et préférentiellement allant de 5 % à 7 % en poids.

Les pigments enrobés hydrophobes présents dans l'émulsion selon l'invention sont des pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

Les pigments destinés à être enrobés peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de

titane ou d'oxychlorure de bismuth, les pigments nacrés colorés tels que le mica titane avec des oxy des de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.
On utilise de préférence des pigments d'oxydes de fer ou de dioxyde de titane.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoro-propylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédement désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les pigments enrobés hydrophobes peuvent être présents en une teneur allant de 0,5 % à 20 % en poids, par rapport au poids total de l'émulsion, de préférence en une teneur au moins égale à 5 % en poids, notamment allant de 5 % à 20 % en poids (notamment allant de 8 % à 20 % en poids), et préférentiellement allant de 8 à 15% en poids.

La phase grasse de l'émulsion selon l'invention comprend au moins 30 % en poids, par rapport au poids total de l'émulsion, de phase grasse volatile comprenant, voire constituée de, un mélange d'huiles volatiles tel que défini précédemment, notamment de 30 % à 45 % en poids, de préférence de 30 % à 40 % en poids, et préférentiellement de 33 % à 38 % en poids. En particulier, le mélange d'huiles volatiles tel que défini précédemment peut être présent en une teneur allant de 30 % à 45 % en poids, de préférence de 30 % à 40 % en poids, et préférentiellement de 33 % à 38 % en poids, par rapport au poids total de l'émulsion.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et de préférence allant de 170 °C à 250 °C.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

L'huile volatile hydrocarbonée utilisable dans l'invention peut être choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars® ou de Permetyls®, les esters ramifiés en $C_8$-$C_{16}$ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile hydrocarbonée peut être présente en une teneur allant de 6 % à 25 % en poids, par rapport au poids total de l'émulsion, de préférence allant de 10 % à 20 % en poids, et préférentiellement allant de 10 % à 15 % en poids. En particulier, la composition comprend au moins 10 % en poids, par rapport au poids total de l'émulsion, d'huile volatile hydrocarbonée.

L' huile volatile siliconée utilisable dans l'invention peut être chpoisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

Comme huile volatile siliconée, on peut citer les huiles de silicones linéaires ou cycliques ayant de 2 à 7

atomes de silicium, ces silicones comportant éventuellement des groupes alkyle ou alkoxy ayant de 1 à 10 atomes de carbone. Comme exemple d'huile volatile siliconée, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

L'huile volatile choisie parmi les huiles volatiles siliconées, les huiles volatiles fluorées, et leurs mélanges peut être présente en une teneur allant de 20 % à 32 % en poids, par rapport au poids total de l'émulsion, de préférence allant de 20 % à 30 % en poids, et préférentiellement allant de 22 % à 26 % en poids. Avantageusement, la phase grasse volatile comprend au moins une huile volatile hydrocarbonée et au moins une huile volatile siliconée.

Selon un mode préfére de réalisation de l'invention, la phase grasse volatile de l'émulsion comprend :

- une première huile volatile hydrocarbonée,
- une deuxième huile volatile siliconée ayant un point éclair supérieur à 55 °C et inférieur ou égal à 80 °C , de préférence allant de 65 °C à 80 °C, et mieux allant de 67°C à 85 °C,
- une troisième huile volatile siliconée ayant un point éclair supérieur à 80 °C, de préférence supérieur ou égal à 80 °C et inférieur ou égal à 95 °C, et mieux allant de 87 °C à 95 °C.

Dans ce mode de réalisation :

- la première huile volatile hydrocarbonée peut être une isoparaffine et en particulier l'isododécane ;
- la deuxième huile volatile siliconée peut être le décaméthyl cyclopentasiloxane, le décaméthyltétrasiloxane, et de préférence le décaméthyl cyclopentasiloxane ;
- la troisième huile volatile siliconée peut être le dodécaméthyl cyclohexasiloxane.

Avantageusement, la première huile volatile hydrocarbonée, et notamment l'isododécane, peut être présente en une teneur allant de 6 % à 25 % en poids, par rapport au poids total de l'émulsion, de préférence allant de 10 % à 20 % en poids, et préférentiellement allant de 10 % à 15 % en poids.

Avantageusement, la deuxième huile volatile siliconée, et notamment le décaméthyl cyclopentasiloxane, peut être présente en une teneur allant de 0,1 % à 31,9 % en poids, par rapport au poids total de l'émulsion, de préférence allant de 5 % à 20 % en poids, et préférentiellement allant de 8 % à 16 % en poids.

Avantageusement, la troisième huile volatile siliconée, et notamment le dodécaméthyl cyclohexasiloxane, peut être présente en une teneur allant de 0,1 % à 31,9 % en poids, par rapport au poids total de l'émulsion, de préférence allant de 5 % à 20 % en poids, et préférentiellement allant de 8 % à 16 % en poids.

Un mode de réalisation particulièrement préféré de la composition selon l'invention est un fond de teint dont la phase grasse volatile comprend au moins 30 % en poids, par rapport au poids total de l'émulsion, d'un mélange de décaméthyl cyclopentasiloxane, de dodécaméthyl cyclohexasiloxane et d'isododécane, la teneur en isododécane étant d'au moins 6 % en poids, par rapport au poids total de l'émulsion, et de préférence d'au moins 10 % en poids.

La phase grasse de l'émulsion selon l'invention peut comprendre en outre au moins une huile additionnelle non volatile.

L'huile additionnelle peut être présente en une teneur allant de 0,1 % à 12 % en poids, par rapport au poids total de l'émulsion, et de préférence allant de 1 % à 5 % en poids.

L'émulsion comprend avantageusement de 30 % à 45 % en poids, par rapport au poids total de l'émulsion, d'huiles , et de préférence de 30 % à 40 % en poids.

L'huile additionnelle non volatile peut être choisie parmi les huiles d'origine minérale, animale, végétale ou synthétique, carbonées, hydrocarbonées et/ou siliconées, et leurs mélanges, dans la mesure où elles sont compatibles avec l'utilisation envisagée. En particulier, l'huile additionnelle peut être choisie parmi les huiles hydrocarbonées non volatiles, les huiles siliconées non volatiles, et leurs mélanges.

On peut citer les huiles hydrocarbonées non volatiles telles que l'huile de paraffine ou de vaseline, l'isoeicosane, l'huile de vison, de tortue, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique,

l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol.

Comme huile non volatile siliconée additionnelle, on peut citer les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, et leurs mélanges.

La phase grasse peut également comprendre au moins une cire, au moins une gomme et/ou au moins un corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Micro-emulsions Theory and Practice", L. M. Prince Ed., Academic Press (1977), pages 21-32. Comme cire liquide à température ambiante, on peut citer l'huile de Jojoba.

Les cires peuvent être présentes à raison de 0,1 % à 10 %, en poids, par rapport au poids total de l'émulsion, et de préférence de 0,1 % à 5 % en poids.

On peut définir les composés gras pâteux à l'aide d'au moins une des propriétés physico-chimiques suivantes :

- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Les compositions de l'invention peuvent également comprendre au moins une alkyl, alcoxy ou phényl-diméthicone telle que, par exemple le produit vendu sous la dénomination de "Abil® wax 2440" par la Société GOLDSCHMIDT.

Les compositions selon l'invention peuvent également comprendre au moins une résine de silicone comprenant une combinaison des unités $R_3SiO_{1/2}$ , $R_2SiO_{2/2}$ , $RSiO_{3/2}$ et $SiO_{4/2}$, dans lesquelles R désigne un radical alkyle ayant de 1 à 6 atomes de carbone.

L'émulsion selon l'invention peut comprendre, en outre, un agent épaississant de la phase grasse. L'agent épaississant peut être choisi parmi :

- les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34®" par la société RHEOX, les hectorites organomodifiées telles que celles vendues sous la dénomination "Bentone 27®", "Bentone 38®" par la société RHEOX.
- la silice pyrogénée hydrophobe, qui est une silice pyrogénée modifiée chimiquement en surface par réaction chimique générant une diminution du nombre de groupes silanol. On peut notamment substituer des groupes silanol par des groupements hydrophobes.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

L'agent épaississant de la phase grasse peut être présent en une teneur allant de 0,1% à 5% en poids, par rapport au poids total de l'émulsion, et mieux de 0,4% à 3% en poids.

La phase grasse peut représenter de 30 % à 45 %, de préférence de 35 % à 45 %, en poids, par rapport au poids total de l'émulsion.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants autres que l'eau comme par exemple les alcools primaires tels que l'éthanol et l'isopropanol, les glycols tels que le propylène glycol, le butylène glycol, le dipropylène glycol, le diéthylène glycol, les éthers de glycol tel que les alkyl($C_1$-$C_4$)éther de mono, di- ou tripropylène glycol, mono, di- ou triéthylène glycol, et leurs mélanges.

La phase aqueuse peut comprendre en outre des agents de stabilisation , par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

De préférence, la phase aqueuse est présente dans l'émulsion selon l'invention en une teneur allant 30 % à 50 %, en poids, de préférence allant de 35 % à 45 % en poids, par rapport au poids total de l'émulsion.

L'émulsion selon l'invention peut comprendre des charges. Par charges, il faut comprendre des particules incolores ou blanches, minérales ou de synthèse, lamellaires ou non lamellaires.

Les charges peuvent être présentes dans l'émulsion en une teneur allant de 0,1 % à 10 % en poids, par rapport au poids total de l'émulsion, de préférence 0,1 % à 7 %. On peut citer notamment le talc, le mica, la silice, le kaolin, l'amidon, le nitrure de bore, le carbonate de calcium, le carbonate ou l'hydrocarbonate de magnésium, la cellulose microcristalline, les poudres de polymères synthétiques tels que le polyéthylène, les polyesters, les polyamides tels que ceux vendus sous la dénomination commerciale de "Nylon", le polytétrafluoroéthylène ("Téflon®") et les poudres de silicone.

Avantageusement, l'émulsion selon l'invention peut avoir une viscosité, mesurée à 25 °C, à une vitesse de cisaillement de 200 $min^{-1}$ (200 tours par minute, soit une fréquence de 50 Hz), allant de 0,15 à 0,6 Pa.s (1,5 à 6 poises), et de préférence allant de 0,25 à 0,45 Pa.s (2,5 à 4,5 poises). Une telle viscosité permet une application facile l'émulsion et d'obtenir un maquillage homogène, uniforme et sans traces. La viscosité est mesurée à 25 °C avec un viscosimètre CONTRAVES type TV équipé d'un mobile n°2, la mesure étant effectuée après 10 minutes de rotation du mobile (temps au bout duquel on observe une stabilisation de la viscosité et de la vitesse de rotation du mobile), à un cisaillement de 200 $min^{-1}$.

De façon connue, toutes les compositions de l'invention peuvent contenir un ou plusieurs des adjuvants habituels dans les domaines cosmétique et dermatologique, des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des agents filmogènes ; des colorantes solubles ; et leurs mélanges. Les quantités de ces différents adjuvants sont celles classiquement utilisées dans les fonds de teint.

Comme actifs utilisables dans la composition de l'invention, on peut citer par exemple les agents hydratants tels que les hydrolysats de protéines et les polyols comme la glycérine, les glycols comme les polyéthylène glycols, et les dérivés de sucre ; les extraits naturels ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les enzymes ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés; les agents tenseurs; et leurs mélanges.

Les filtres solaires (ou filtres U.V.) peuvent être choisis parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.
Comme filtres UVB, on peut citer par exemple :

(1) les dérivés de l'acide salicylique, en particulier le salicylate d'homomenthyle et le salicylate d'octyle ;

(2) les dérivés de l'acide cinnamique, en particulier le p-méthoxycinnamate de 2-éthylhexyle, commercialisé par

la société Givaudan sous la dénomination Parsol MCX® ;

(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539® ;

(4) les dérivés de l'acide p-aminobenzoïque ;

(5) le 4-méthyl benzylidène camphre commercialisé par la société Merck sous la dénomination EUSOLEX 6300® ;

(6) l'acide 2-phénylbenzimidazole 5-sulfonique commercialisé sous la dénomination EUSOLEX 232® par la société Merck ;

(7) les dérivés de 1,3,5-triazine, en particulier :

- la 2,4,6-tris[p-(2'-éthylhexyl-1'-oxycarbonyl)anilino]-1,3,5-triazine commercialisé par la société BASF sous la dénomination UVINUL T150®, et
- le dioctyl butamido triazone commercialisé par la société Sigma 3V sous la dénomination UVASORB HEB® ;

(8) les mélanges de ces filtres.

Comme filtres UVA, on peut citer par exemple :

(1) les dérivés de dibenzoylméthane, en particulier le 4-(ter.-butyl) 4'-méthoxy dibenzoylméthane commercialisé par la société Givaudan sous la dénomination PARSOL 1789® ;

(2) l'acide benzène 1,4 [di(3-méthylidènecampho-10-sulfonique)] éventuellement sous forme partiellement ou totalement neutralisée, commercialisé sous la dénomination MEXORYL SX® par la société Chimex.

(3) les dérivés de benzophénone, par exemple :

- la 2,4-dihydroxybenzophénone (benzophénone-1) ;
- la 2,2',4,4'-tétra-hydroxybenzophénone (benzophénone-2) ;
- la 2-hydroxy-4-méthoxy-benzophénone (benzophénone-3), commercialisé sous la dénomination UVINUL M40® par la société BASF ;
- l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40® ;
- la 2,2'-dihydroxy-4,4'-diméthoxy-benzophénone (benzophénone-6) ;
- la 5-chloro-2-hydroxybenzophénone (benzophénone-7) ;
- la 2,2'-dihydroxy-4-méthoxy-benzophénone (benzophénone-8) ;
- le sel disodique du diacide 2,2'-dihydroxy-4,4'-diméthoxy-benzophénone-5,5'-disulfonique (benzophénone-9) ;
- la 2-hydroxy-4-méthoxy-4'-méthyl-benzophénone (benzophénone-10) ;
- la benzophénone-11 ;
- la 2-hydroxy-4-(octyloxy)benzophénone (benzophénone-12).

(4) les dérivés silanes ou les polyorganosiloxanes à groupement benzophénone ;

(5) les anthranilates, en particulier l'anthranilate de menthyle commercialisé par la société Haarman & Reiner sous la dénomination NEO HELIOPAN MA® ;

(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer;

(7) les dérivés siliciés de benzimidazolyl-benzazoles N-substitués ou de benzofuranyl-benzazoles, et en particulier :

- le 2-[1-[3-[1,3,3,3-tétraméthyl-1-[(triméthylsilyl)oxy]disiloxanyl]propyl]-1H-benzimidazol-2-yl]-benzoxazole ;

- le 2-[1-[3-[1,3,3,3-tétraméthyl-1-[(triméthylsilyl)oxy]disiloxanyl]propyl]-1H-benzimidazol-2-yl]-benzothiazole ;
- le 2-[1-(3-trimethylsilanyl-propyl)-1H-benzimidazol-2-yl]-benzoxazole ;
- le 6-méthoxy-1,1'-bis-(3-triméthylsilanyl-propyl)-1H,1'H-[2,2']bibenzimidazolyl-benzoxazole ;
- le 2-[1-(3-trimethylsilanyl-propyl)-1H-benzimidazol-2-yl]-benzothiazole ; qui sont décrits dans la demande de brevet EP-A-1 028 120 ;

(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S®, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M® ;

(9) leurs mélanges.

On peut aussi utiliser un mélange de plusieurs de ces filtres et un mélange de filtres UVB et de filtres UVA et aussi des mélanges avec des filtres physiques.
Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm.
Ces pigments sont de préférence traités de manière à rendre leur surface hydrophobe; ce traitement peut être effectué selon les méthodes connues de l'homme du métier ; les pigments peuvent notamment être enrobés par des composés siliconés tels que des PDMS et/ou par des polymères.
Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.
L'invention est illustrée plus en détail dans les exemples suivants.

**Exemple 1 :**

On a préparé un fond de teint sous forme d'émulsion eau-dans-huile ayant la composition suivante :

| Phase huileuse | |
|---|---|
| Isododécane | 13 g |
| Cyclopentasiloxane | 16 g |
| Cyclohexasiloxane | 8 g |
| Polydiméthylsiloxane (DC 200 Fluid - 5 cst de la société DOW CORNING) | 2 g |
| Isoeicosane | 3 g |
| Cetyl diméthicone copolyol (Abil® EM 90 de la société GOLDSCHMIDT) | 0,8 g |
| Diméthicone copolyol (KF6017 de Shin Etsu) | 5 g |
| Isostéarate de polyglycérol (4 moles de glycérol) | 0,6 g |
| Hectorite | 1,4 g |
| Oxydes de fer enrobés de perfluoroalkylphosphate | 2 g |
| Oxyde de titane enrobé de perfluoroalkylphosphate | 5,5 g |
| Poudre de nylon | 4 g |

| Phase aqueuse | |
|---|---|
| Butylène glycol | 10 g |
| Chlorure de sodium | 0,7 g |
| Conservateurs | |
| Eau qsp | 100 g |

L'émulsion est préparée à température ambiante d'une part en mélangeant les pigments dans une partie du cyclopentasiloxane, d'autres part en mélangeant les autres huiles avec les tensioactifs, puis on ajoute le mélange de

pigments et le nylon aux autres constituants de la phase grasse mélangés. On prépare ensuite le mélange des constituants de la phase aqueuse que l'on verse dans le mélange de la phase grasse, sous agitation selon les moyens connus pour obtenir au final l'émulsion.

Ce fond de teint est stable après stockage à température ambiante (25 °C) pendant 4 mois. Il s'applique facilement sur la peau avec une bonne sensation d'onctuosité et de douceur, un très bon glissant ; il sèche rapidement après l'application du produit, et le maquillage obtenu présente une bonne homogénéité de la couleur, sans laisser de trace sur la peau.

**Exemple 2 :**

On a préparé un fond de teint sous forme d'émulsion eau-dans-huile ayant la composition suivante :

| Phase huileuse | |
|---|---|
| Isododécane | 13 g |
| Cyclopentasiloxane | 16 g |
| Cyclohexasiloxane | 6,8 g |
| Polydiméthylsiloxane (DC 200 Fluid - 5 cst de la société DOW CORNING) | 2 g |
| Isoeicosane | 3 g |
| Cetyl diméthicone copolyol (Abil® EM 90 de la société GOLDSCHMIDT) | 2 g |
| Diméthicone copolyol (KF6017 de Shin Etsu) | 5 g |
| Isostéarate de polyglycérol (4 moles de glycérol) | 0,6 g |
| Hectorite | 1,4 g |
| Oxydes de fer enrobés de perfluoroalkylphosphate | 2 g |
| Oxyde de titane enrobé de perfluoroalkylphosphate | 5,5 g |
| Poudre de nylon | 4g |

| Phase aqueuse | |
|---|---|
| Butylène glycol | 10 g |
| Chlorure de sodium | 0,7 g |
| Conservateurs | |
| Eau qsp | 100 g |

Le fond de teint présente une bonne stabilité à température ambiante. Il s'applique facilement sur la peau sans sensation de gars ; il séche rapidement après l'application du produit sur la peau ; le maquillage obtenu présente une bonne homogénéité de la couleur, sans laisser de trace sur la peau

## Revendications

**1.** Fond de teint sous forme d'émulsion eau-dans-huile comprenant une phase grasse, une phase aqueuse, un alkyl $C_8$-$C_{22}$ diméthicone copolyol, un diméthicone copolyol présent en une teneur allant de 5 % à 10 % en poids, par rapport au poids total de l'émulsion, des pigments enrobés hydrophobes, la phase grasse comprenant au moins 30 % en poids, par rapport au poids total de l'émulsion, de phase grasse volatile comprenant :

- au moins 6 % en poids, par rapport au poids total de l'émulsion, d' au moins une huile volatile hydrocarbonée, et
- au moins une huile volatile choisie parmi les huiles volatiles siliconées, les huiles volatiles fluorées, et leurs mélanges.

**2.** Fond de teint selon la revendication 1, **caractérisé par le fait que** l'alkyl $C_8$-$C_{22}$ diméthicone copolyol est un composé de formule (I) suivante :

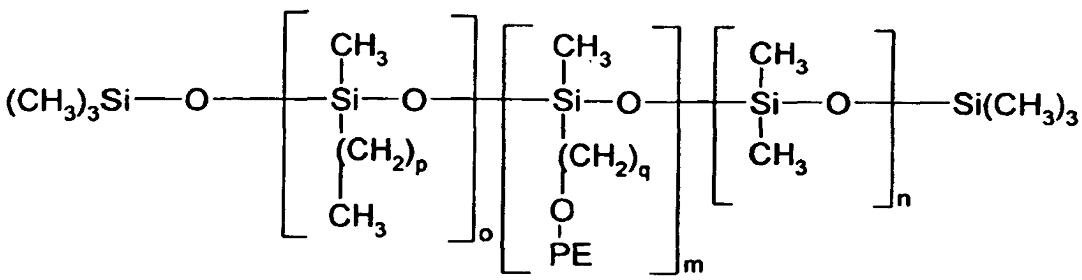

(I)

dans laquelle :

- PE représente $(-C_2H_4O)_x-(C_3H_6O)_y-R$, R étant choisi parmi un atome d'hydrogène et un radical alkyle de 1 à 4 atomes de carbone, x allant de 0 à 100 et y allant de 0 à 80, x et y n'étant pas simultanément 0
- m va de 1 à 40
- n va de 10 à 200
- o va de 1 à 100
- p va de 7 et 21
- q va de 0 à 4

**3.** Fond de teint selon la revendication 2, **caractérisé par le fait que** R = H ; m = 1 à 10 ; n = 10 à 100 ; o = 1 à 30 ; p = 15 ; q = 3

**4.** Fond de teint selon l'une quelconque des revendications précédentes, **caractérisé par le fait que** l'alkyl $C_8$-$C_{22}$ diméthicone copolyol est le cétyl diméthicone copolyol.

**5.** Fond de teint selon l'une quelconque des revendications précédentes, **caractérisé par le fait que** l'alkyl $C_8$-$C_{22}$ diméthicone copolyol est présent en une teneur allant de 0,5 % à 2 % en poids, par rapport au poids total de l'émulsion, notamment allant de 0,6 % à 2 % en poids, mieux allant de 0,7 % à 2 % en poids, voire allant de 0,8 % à 2 % en poids et de préférence allant de 0,5 % à 1,5 % en poids, notamment allant de 0,6 % à 1,5 % en poids, mieux allant de 0,7 % à 1,5 % en poids, voire allant de 0,8 % à 1,5 % en poids.

**6.** Fond de teint selon l'une quelconque des revendications précédentes,**caractérisé par le fait que** le diméthicone copolyol est un composé de formule (II) suivante :

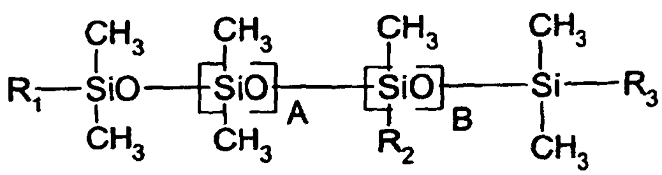

(II)

dans laquelle:

$R_1$, $R_2$, $R_3$, indépendamment les uns des autres, représentent un radical alkyle en $C_1$-$C_6$ ou un radical $-(CH_2)_x-(OCH_2CH_2)_y-(OCH_2CH_2CH_2)_z-OR_4$, au moins un radical $R_1$, $R_2$ ou $R_3$ n'étant pas un radical alkyle ; $R_4$ étant un hydrogène, un radical alkyle en $C_1$-$C_3$ ou un radical acyle en $C_2$-$C_4$ ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

**7.** Fond de teint selon la revendication 6, **caractérisé par le fait que** $R_1 = R_3$ = radical méthyle, x est un nombre entier allant de 2 à 6 et y est un nombre entier allant de 4 à 30.

**8.** Fond de teint selon la revendication 6 ou 7, **caractérisé par le fait que** $R_4$ est un hydrogène.

**9.** Fond de teint selon l'une quelconque des revendications 1 à 6, **caractérisé par le fait que** le diméthicone copolyol est un composé de formule (III) suivante :

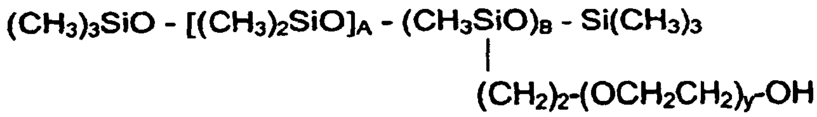

(III)

dans laquelle A est un nombre entier allant de 20 à 105, B est un nombre entier allant de 2 à 10 et y est un nombre entier allant de 10 à 20.

**10.** Fond de teint selon l'une quelconque des revendications 1 à 6, **caractérisé par le fait que** le diméthicone copolyol est un composé de formule (IV) suivante :

$$HO - (CH_2CH_2O)_y\text{-}(CH_2)_3 - [(CH_3)_2SiO]_{A'} - [(CH_3)_2Si] - (CH_2)_3 - (OCH_2CH_2)_y - OH \qquad (IV)$$

dans laquelle A' et y sont des nombres entiers allant de 10 à 20.

**11.** Fond de teint selon l'une quelconque des revendications précédentes, **caractérisé par le fait que** le diméthicone copolyol est présent en une teneur allant de 5 % à 8 % en poids, par rapport au poids total de l'émulsion, de préférence allant de 5 % à 7 % en poids.

**12.** Fond de teint selon l'une quelconque des revendications précédentes, **caractérisé par le fait que** les pigments enrobés hydrophobes sont choisis parmi les oxydes métalliques, le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés, et leurs mélanges.

**13.** Fond de teint selon l'une quelconque des revendications précédentes, **caractérisé par le fait que** les pigments enrobés hydrophobes sont choisis parmi les oxydes de fer et les dioxydes de titane.

**14.** Fond de teint selon l'une quelconque des revendications précédentes, **caractérisé par le fait que** les pigments enrobés hydrophobes sont traités avec un agent hydrophobe choisi parmi les silicones, les acides gras, les savons métalliques, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

**15.** Fond de teint selon la revendication 14, **caractérisé par le fait que** les acides aminés N-acylés comprennent un groupe acyle ayant de 8 à 22 atomes de carbones.

**16.** Fond de teint selon l'une quelconque des revendications précédentes, **caractérisé par le fait que** les pigments enrobés hydrophobes sont présents en une teneur allant de 0,5 % à 20 % en poids, par rapport au poids total de l'émulsion, de préférence en une teneur au moins égale à 5 % en poids, et préférentiellement allant de 5 % à 20 % en poids.

**17.** Fond de teint selon l'une quelconque des revendications précédentes, **caractérisé par le fait que** la phase grasse volatile est présente en une teneur allant de 30 % à 45 % en poids, par rapport au poids total de l'émulsion, de préférence allant de 30 % à 40 % en poids, et préférentiellement allant de 33 % à 38 % en poids.

**18.** Fond de teint selon l'une quelconque des revendications précédentes, **caractérisé par le fait que** l'huile volatile hydrocarbonée est choisie parmi les huiles hydrocarbonées ayant un point éclair allant de 40 °C à 102 °C, de préférence allant de 40 °C à 55 °C, et préférentiellement allant de 40 °C à 50 °C.

**19.** Fond de teint selon l'une quelconque des revendications précédentes, **caractérisé par le fait que** l'huile volatile hydrocarbonée est choisie parmi les huiles volatiles hydrocarbonées ayant de 8 à 16 atomes de carbone et leurs mélanges.

**20.** Fond de teint selon l'une quelconque des revendications précédentes, **caractérisé par le fait que** l'huile volatile hydrocarbonée est choisie parmi les alcanes ramifiés en $C_8$-$C_{16}$, les esters ramifiés en $C_8$-$C_{16}$, et leurs mélanges.

**21.** Fond de teint selon l'une quelconque des revendications précédentes, **caractérisé par le fait que** l'huile volatile hydrocarbonée est choisie parmi l'isododécane, l'isodécane, l'isohexadécane.

**22.** Fond de teint selon l'une quelconque des revendications précédentes, **caractérisé par le fait que** l'huile volatile hydrocarbonée est l'isododécane.

**23.** Fond de teint selon l'une quelconque des revendications précédentes, **caractérisée par le fait que** l'huile volatile hydrocarbonée est présente en une teneur allant de 6 % à 25 % en poids, par rapport au poids total de l'émulsion, de préférence allant de 10 % à 20 % en poids, et préférentiellement allant de 10 % à 15 % en poids.

**24.** Fond de teint selon l'une quelconque des revendications précédentes, **caractérisé par le fait que** l'huile volatile siliconée est choisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

**25.** Fond de teint selon l'une quelconque des revendications précédentes, **caractérisée par le fait que** l'huile volatile siliconée est choisie parmi les huiles de silicones linéaires ou cycliques ayant de 2 à 7 atomes de silicium, ces silicones comportant éventuellement des groupes alkyle où alkoxy ayant de 1 à 10 atomes de carbone.

**26.** Fond de teint selon l'une quelconque des revendications précédentes, **caractérisé par le fait que** l'huile volatile siliconée est choisie parmi l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

**27.** Fond de teint selon l'une quelconque des revendications précédentes, **caractérisé par le fait que** l'huile volatile fluorée est choisie parmi le nonafluoroéthoxybutane, le nonafluorométhoxybutane, le décafluoropentane, le tétradécafluorohexane, le dodécafluoropentane, et leurs mélanges.

**28.** Fond de teint selon l'une quelconque des revendications précédentes, **caractérisé par le fait que** la phase grasse volatile comprend au moins une huile volatile hydrocarbonée et au moins une huile volatile siliconée.

**29.** Fond de teint selon l'une quelconque des revendications précédentes, **caractérisé par le fait que** l'huile volatile choisie parmi les huiles volatiles siliconées, les huiles volatiles fluorées, et leurs mélanges est présente en une teneur allant de 20 % à 32 % en poids, par rapport au poids total de émulsion, de préférence allant de 20 % à 30 % en poids, et préférentiellement allant de 22 % à 26 % en poids.

**30.** Fond de teint selon la revendication précédente, **caractérisé par le fait que** la phase grasse volatile comprend :

- une première huile volatile hydrocarbonée,
- une deuxième huile volatile siliconée ayant un point éclair supérieur à 55 °C et inférieur ou égal à 80 °C , de préférence allant de 65 °C à 80 °C, et mieux allant de 67 °C à 85 °C,
- une troisième huile volatile siliconée ayant un point éclair supérieur à 80 °C, de préférence supérieur ou égal à 80 °C et inférieur ou égal à 95 °C, et mieux allant de 87 °C à 95 °C.

**31.** Fond de teint selon la revendication précédente, **caractérisé par le fait que** la première huile volatile hydrocarbonée est l'isododécane.

**32.** Fond de teint selon la revendication 30, **caractérisé par le fait que** la deuxième huile siliconée est choisie parmi le décaméthyl cyclopentasiloxane, le décaméthyltétrasiloxane, et de préférence le décaméthyl cyclopentasiloxane.

**33.** Fond de teint selon la revendication 30, **caractérisé par le fait que** la troisième huile siliconée est le dodécaméthyl cyclohexasiloxane.

**34.** Fond de teint selon l'une quelconque des revendications 30 à 33, **caractérisé par le fait que** la deuxième huile volatile siliconée est présente en une teneur allant de 0,1 % à 31,9 % en poids, par rapport au poids total de l'émulsion, de préférence allant de 5 % à 20 % en poids, et préférentiellement allant de 8 % à 16 % en poids.

**35.** Fond de teint selon l'une quelconque des revendications 30 à 34, **caractérisé par le fait que** la troisième huile volatile siliconée est présente en une teneur allant de 0,1 % à 31,9 % en poids, par rapport au poids total de l'émulsion, de préférence allant de 5 % à 20 % en poids, et préférentiellement allant de 8 % à 16 % en poids.

**36.** Fond de teint selon l'une quelconque des revendicationsprécédentes, **caractérisé par le fait que** la phase grasse volatile comprend au moins 30 % en poids, par rapport au poids total de l'émulsion, d'un mélange de décaméthyl cyclopentasiloxane, de dodécaméthyl cyclohexasiloxane et d'isododécane, la teneur en isododécane étant d'au moins 6 % en poids, par rapport au poids total de l'émulsion.

**37.** Fond de teint selon la revendication 36, **caractérisé par le fait que** la teneur en isododécane est d'au moins 10 % en poids, par rapport au poids total de l'émulsion.

**38.** Fond de teint selon l'une quelconque des revendications précédentes, **caractérisé par le fait qu'**il comprend une huile additionnelle non volatile.

**39.** Fond de teint selon la revendication 38, **caractérisé par le fait que** l'huile additionnelle est choisie parmi les huiles hydrocarbonées non volatiles, les huiles siliconées non volatiles, et leurs mélanges.

**40.** Fond de teint selon la revendication 38 ou 39, **caractérisé par le fait que** l'huile additionnelle est présente en une teneur allant de 0,1 % à 12 % en poids, par rapport au poids total de l'émulsion, et de préférence allant de 1 % à 5 % en poids.

**41.** Fond de teint selon l'une quelconque des revendications précédentes, **caractérisé par le fait qu'**il comprend des huiles en une teneur allant de 30 % à 45 % en poids, par rapport au poids total de l'émulsion, et de préférence allant de 30 % à 40 % en poids.

**42.** Fond de teint selon l'une quelconque des revendications précédentes, **caractérisé par le fait qu'**il comprend un corps gras choisi parmi les cires, les gommes, les corps gras pâteux, et leurs mélanges.

**43.** Fond de teint selon l'une quelconque des revendications précédentes, **caractérisé par le fait qu'**il comprend un agent épaississant de la phase grasse.

**44.** Fond de teint selon la revendication 43, **caractérisé par le fait que** l'agent épaississant est choisi parmi les argiles organomodifiées et la silice pyrogénée hydrophobe.

**45.** Fond de teint selon la revendication 43 ou 44, **caractérisé par le fait que** l'agent épaississant de la phase grasse est présent en une teneur allant de 0,1% à 5% en poids, par rapport au poids total de l'émulsion, et mieux de 0,4% à 3% en poids.

**46.** Fond de teint selon l'une quelconque des revendications précédentes, **caractérisé par le fait que** la phase grasse représente de 22 % à 50 % en poids, par rapport au poids total de l'émulsion, de préférence de 25 % à 45 % en poids, et préférentiellement de 30 % à 40 % en poids.

**47.** Fond de teint selon l'une quelconque des revendications précédentes, **caractérisé par le fait que** la phase aqueuse est présente en une teneur allant 30 % à 50 %, en poids, de préférence allant de 35 % à 45 % en poids, par rapport au poids total de l'émulsion.

**48.** Fond de teint selon l'une quelconque des revendications précédentes, **caractérisé par le fait que** la phase aqueuse comprend de l'eau et/ou un solvant choisi parmi les alcools primaires, les glycols, les éthers de glycol, et leurs mélanges et/ou un agent de stabilisation.

**49.** Fond de teint selon l'une quelconque des revendications précédentes, **caractérisé par le fait qu'**il comprend des charges.

**50.** Fond de teint selon la revendication 49, **caractérisé par le fait que** les charges sont choisies parmi le talc, le mica, la silice, le kaolin, l'amidon, le nitrure de bore, le carbonate de calcium, le carbonate ou l'hydrocarbonate de magnésium, la cellulose microcristalline, les poudres de polyéthylène, les polyesters, les polyamides, le polytétrafluoroéthylène les poudres de silicone, et leurs mélanges.

**51.** Fond de teint selon la revendication 49 ou 50, **caractérisé par le fait que** les charges sont présentes en une teneur allant de 0,1 % à 15 % en poids, par rapport au poids total de l'émulsion, de préférence 0,1 % à 10 %.

**52.** Fond de teint selon l'une quelconque des revendications précédentes, **caractérisé par le fait qu'**il comprend en outre au moins un additif choisi parmi les agents gélifiants, les épaississants hydrophiles ou lipophiles, les agents hydratants ; les émollients ; les actifs hydrophiles ou lipophiles ; les agents anti-radicaux libres ; les séquestrants ; les antioxydants ; les conservateurs ; les agents alcanisants ou acidifiants ; les parfums ; les agents filmogènes ; les colorants solubles, et leurs mélanges.

**53.** Fond de teint selon l'une quelconque des revendications précédentes, **caractérisé par le fait qu'**il a une viscosité , mesurée à 25 °C, à une vitesse de cisaillement de 200 $min^{-1}$, allant de 0,15 à 0,6 Pa.s, et de préférence allant de 0,25 à 0,45 Pa.s.

**54.** Procédé cosmétique de maquillage non thérapeutique de la peau comprenant l'application sur la peau d'un fond de teint selon l'une quelconque des revendications précédentes.

**55.** Utilisation d'un fond de teint selon l'une quelconque des revendications 1 à 53 pour obtenir un maquillage homogène de la peau.

**56.** Utilisation d'un alkyl $C_8$-$C_{22}$ diméthicone copolyol et d'un diméthicone copolyol dans une composition de fond de teint sous forme d'émulsion eau-dans-huile contenant une phase grasse, une phase aqueuse, des pigments enrobés hydrophobes, la phase grasse comprenant au moins 30 % en poids, par rapport au poids total de l'émulsion, de phase grasse volatile comprenant :

- au moins 6 % en poids, par rapport au poids total de l'émulsion, d' au moins une huile volatile hydrocarbonée, et
- au moins une huile volatile choisie parmi les huiles volatiles siliconées, les huiles volatiles fluorées, et leurs mélanges, le diméthicone copolyol étant présent en une teneur allant de 5 % à 10 % en poids, par rapport au poids total de l'émulsion, pour obtenir une émulsion stable et/ou homogène et/ou pour obtenir un maquillage homogène de la peau.

## Patentansprüche

**1.** Make-up in Form einer Wasser-in-Öl-Emulsion, die eine Fettphase, eine wässrige Phase, ein $C_{8-22}$-Alkyldimethiconcopolyol, ein Dimethiconcopolyol, das in einer Menge von 5 bis 10 Gew.-%, bezogen auf das Gesamtgewicht der Emulsion, enthalten ist, und umhüllte hydrophobe Pigmente enthält, wobei die Fettphase mindestens 30 Gew.-% flüchtige Fettphase, bezogen auf das Gesamtgewicht der Emulsion, enthält, welche umfasst:

- mindestens 6 Gew.-% mindestens eines flüchtigen Kohlenwasserstofföls, bezogen auf das Gesamtgewicht der Emulsion, und
- mindestens ein flüchtiges Öl, das unter den flüchtigen Siliconölen, den flüchtigen fluorierten Ölen und deren Gemischen ausgewählt ist.

**2.** Make-up nach Anspruch 1, **dadurch gekennzeichnet, dass** das $C_{8-22}$-Alkyldimethiconcopolyol eine Verbindung der folgenden Formel (I) ist:

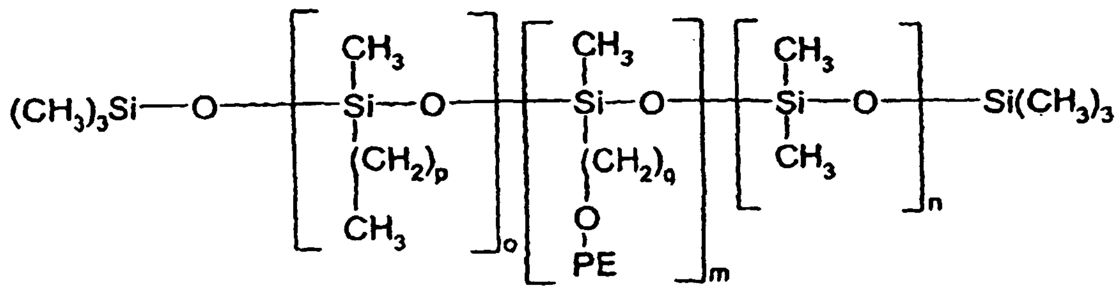

(I),

wobei in der Formel:

- PE $(-C_2H_4O)_x-(C_3H_6O)_y-R$ bedeutet, wobei R ein Wasserstoffatom oder eine Alkylgruppe mit 1 bis 4 Kohlenstoffatomen ist, x im Bereich von 0 bis 100 liegt und y im Bereich von 0 bis 80 liegt, wobei x und y nicht gleichzeitig 0 sein können,
- m im Bereich von 1 bis 40 liegt,
- n im Bereich von 10 bis 200 liegt,
- o im Bereich von 1 bis 100 liegt,
- p im Bereich von 7 bis 21 liegt, und
- q im Bereich von 0 bis 4 liegt.

**3.** Make-up nach Anspruch 2, **dadurch gekennzeichnet, dass** bedeuten: R = H; m = 1 bis 10; n = 10 bis 100; o = 1 bis 30; p = 15; q=3.

**4.** Make-up nach einem der vorhergehenden Ansprüche, **dadurch gekennzeichnet, dass** das $C_{8-22}$-Alkyldimethiconcopolyol das Cetyldimethiconcopolyol ist.

**5.** Make-up nach einem der vorhergehenden Ansprüche, **dadurch gekennzeichnet, dass** das $C_{8-22}$-Alkyldimethiconcopolyol in einer Menge von 0,5 bis 2 Gew.-%, insbesondere 0,6 bis 2 Gew.-%, besser 0,7 bis 2 Gew.-%, sogar 0,8 bis 2 Gew.-%, vorzugsweise 0,5 bis 1,5 Gew.-%, insbesondere 0,6 bis 1,5 Gew.-%, besser 0,7 bis 1,5 Gew.-% und sogar 0,8 bis 1,5 Gew.-%, bezogen auf das Gesamtgewicht der Emulsion, enthalten ist.

**6.** Make-up nach einem der vorhergehenden Ansprüche, **dadurch gekennzeichnet, dass** das Dimethiconcopolyol eine Verbindung der folgenden Formel (II) ist:

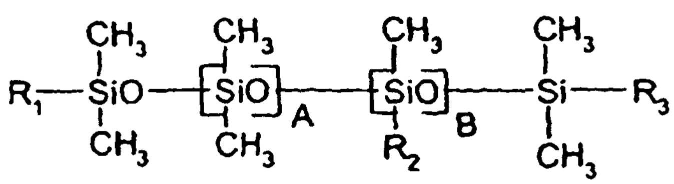

(II),

worin bedeuten:

$R_1$, $R_2$ und $R_3$, unabhängig voneinander $C_{1-6}$-Alkyl oder $-(CH_2)_x-(OCH_2CH_2)_y-(OCH_2CH_2CH_2)_z-OR_4$, wobei mindestens eine der Gruppen $R_1$, $R_2$ oder $R_3$ keine Alkylgruppe bedeutet; wobei $R_4$ Wasserstoff, $C_{1-3}$-Alkyl oder $C_{2-4}$-Acyl bedeutet;
A 0 oder eine ganze Zahl von 1 bis 200;
B 0 oder eine ganze Zahl von 1 bis 50, mit der Maßgabe, dass A und B nicht gleichzeitig 0 sind;
x eine ganze Zahl von 1 bis 6;
y eine ganze Zahl von 1 bis 30; und
z 0 oder eine ganze Zahl von 1 bis 5.

**7.** Make-up nach Anspruch 6, **dadurch gekennzeichnet, dass** bedeuten: $R_1 = R_3$ = Methyl, x eine ganze Zahl von 2 bis 6 und y eine ganze Zahl von 4 bis 30.

**8.** Make-up nach Anspruch 6 oder 7, **dadurch gekennzeichnet, dass** $R_4$ Wasserstoff bedeutet.

**9.** Make-up nach einem der Ansprüche 1 bis 6, **dadurch gekennzeichnet, dass** das Dimethiconcopolyol eine Verbindung der folgenden Formel (III) ist:

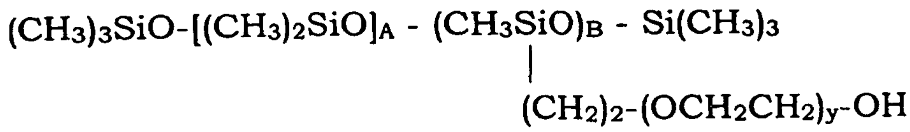

(III),

worin A eine Zahl von 20 bis 105, B eine ganze Zahl von 2 bis 10 ist und y eine ganze Zahl von 10 bis 20 bedeuten.

**10.** Make-up nach einem der Ansprüche 1 bis 6, **dadurch gekennzeichnet, dass** das Dimethiconcopolyol eine Verbindung der folgenden Formel (IV) ist:

$$HO\text{-}(CH_2CH_2O)_y\text{-}(CH_2)_3\text{-}[(CH_3)_2SiO]_{A'}\text{-}[(CH_3)_2Si]\text{-}(CH_2)_3\text{-}(OCH_2CH_2)_y\text{-}OH \qquad (IV)$$

worin A' und y ganze Zahlen von 10 bis 20 bedeuten.

**11.** Make-up nach einem der vorhergehenden Ansprüche, **dadurch gekennzeichnet, dass** das Dimethiconcopolyol in einer Menge von 5 bis 8 Gew.-%, bezogen auf das Gesamtgewicht der Emulsion, und vorzugsweise im Bereich von 5 bis 7 Gew.-% enthalten ist.

**12.** Make-up nach einem der vorhergehenden Ansprüche, **dadurch gekennzeichnet, dass** die umhüllten hydrophoben Pigmente unter den Metalloxiden, Manganviolett, Ultramarinblau, Preußischblau, Ultramarin, Eisenblau, Bismutoxidchlorid, Perlmutt, mit Titan oder Bismutoxidchlorid überzogenen Glimmerpigmenten, farbigen Perlglanzpigmenten und deren Gemischen ausgewählt sind.

**13.** Make-up nach einem der vorhergehenden Ansprüche, **dadurch gekennzeichnet, dass** die umhüllten hydrophoben Pigmente unter den Eisenoxiden und Titandioxid ausgewählt sind.

**14.** Make-up nach einem der vorhergehenden Ansprüche, **dadurch gekennzeichnet, dass** die umhüllen hydrophoben Pigmente mit einem hydrophoben Stoff behandelt sind, der unter den Siliconen, Fettsäuren, Metallsalzen, Perfluoralkylphosphaten, Perfluoralkylsilanen, Perfluoralkylsilazanen, Hexafluorpropylenpolyoxiden, Polyorganosiloxanen, die Perfluoralkylperfluorpolyethergruppen enthalten, Aminosäuren; N-acylierten Aminosäuren oder deren Salzen, Lecithin, Isopropyltriisostearyltitanat und deren Gemischen ausgewählt ist.

**15.** Make-up nach Anspruch 14, **dadurch gekennzeichnet, dass** die N-acylierten Aminosäuren eine Acylgruppe mit 8 bis 22 Kohlenstoffatomen enthalten.

**16.** Make-up nach einem der vorhergehenden Ansprüche, **dadurch gekennzeichnet, dass** die umhüllten hydrophoben Pigmente in einer Menge von 0,5 bis 20 Gew.-%, bezogen auf das Gesamtgewicht der Emulsion, vorzugsweise in einer Menge von mindestens 5 Gew.-% und noch bevorzugter in einer Menge von 5 bis 20 Gew.-% enthalten sind.

**17.** Make-up nach einem der vorhergehenden Ansprüche, **dadurch gekennzeichnet, dass** die flüchtige Fettphase in einer Menge von 30 bis 45 Gew.-%, bezogen auf das Gesamtgewicht der Emulsion, vorzugsweise 30 bis 40 Gew.-% und noch bevorzugter 33 bis 38 Gew.-% enthalten ist.

**18.** Make-up nach einem der vorhergehenden Ansprüche, **dadurch gekennzeichnet, dass** das flüchtige Kohlenwasserstofföl unter den Kohlenwasserstoffölen mit einem Flammpunkt von 40 bis 102 °C, vorzugsweise 40 bis 55 °C und noch bevorzugter 40 bis 50 °C ausgewählt ist.

19. Make-up nach einem der vorhergehenden Ansprüche, **dadurch gekennzeichnet, dass** das flüchtige Kohlenwasserstofföl unter den flüchtigen Kohlenwasserstoffölen mit 8 bis 16 Kohlenstoffatomen und deren Gemischen ausgewählt ist.

20. Make-up nach einem der vorhergehenden Ansprüche, **dadurch gekennzeichnet, dass** das flüchtige Kohlenwasserstofföl unter den verzweigten $C_{8-16}$-Alkanen, verzweigten $C_{8-16}$-Estern und deren Gemischen ausgewählt ist.

21. Make-up nach einem der vorhergehenden Ansprüche, **dadurch gekennzeichnet, dass** das flüchtige Kohlenwasserstofföl unter Isododecan, Isodecan und Isohexadecan ausgewählt ist.

22. Make-up nach einem der vorhergehenden Ansprüche, **dadurch gekennzeichnet, dass** das flüchtige Kohlenwasserstofföl das Isododecan ist.

23. Make-up nach einem der vorhergehenden Ansprüche, **dadurch gekennzeichnet, dass** das flüchtige Kohlenwasserstofföl in einer Menge von 6 bis 25 Gew.-%, bezogen auf das Gesamtgewicht der Emulsion, vorzugsweise 10 bis 20 Gew.-% und noch bevorzugter 10 bis 15 Gew.-% enthalten ist.

24. Make-up nach einem der vorhergehenden Ansprüche, **dadurch gekennzeichnet, dass** das flüchtige Siliconöl unter den Siliconölen mit einem Flammpunkt von 40 bis 102 °C, vorzugsweise einem Flammpunkt über 55 °C und höchstens 95 °C und bevorzugt im Bereich von 65 bis 95 °C ausgewählt ist.

25. Make-up nach einem der vorhergehenden Ansprüche, **dadurch gekennzeichnet, dass** das flüchtige Siliconöl unter den geradkettigen oder cyclischen Siliconen mit 2 bis 7 Siliciumatomen ausgewählt ist, wobei die Silicone gegebenenfalls Alkyl- oder Alkoxygruppe mit 1 bis 10 Kohlenstoffatomen enthalten.

26. Make-up nach einem der vorhergehenden Ansprüche, **dadurch gekennzeichnet, dass** das flüchtige Siliconöl unter Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemischen ausgewählt ist.

27. Make-up nach einem der vorhergehenden Ansprüche, **dadurch gekennzeichnet, dass** das flüchtige fluorierte Öl unter Nonafluorethoxybutan, Nonafluormethoxybutan, Decafluorpentan, Tetradecafluorhexan, Dodecafluorpentan und deren Gemischen ausgewählt ist.

28. Make-up nach einem der vorhergehenden Ansprüche, **dadurch gekennzeichnet, dass** die flüchtige Fettphase mindestens ein flüchtiges Kohlenwasserstofföl und mindestens ein flüchtiges Siliconöl enthält.

29. Make-up nach einem der vorhergehenden Ansprüche, **dadurch gekennzeichnet, dass** das flüchtige Öl, das unter den flüchtigen Siliconölen, den flüchtigen fluorierten Ölen und deren Gemischen ausgewählt ist, in einer Menge von 20 bis 32 Gew.-%, bezogen auf das Gesamtgewicht der Emulsion, vorzugsweise 20 bis 30 Gew.-% und noch bevorzugter 22 bis 26 Gew.-% vorliegt.

30. Make-up nach dem vorhergehenden Anspruch, **dadurch gekennzeichnet, dass** die flüchtige Fettphase enthält:

- ein erstes flüchtiges Kohlenwasserstofföl,
- ein zweites flüchtiges Siliconöl mit einem Flammpunkt über 55 °C und höchsten 80 °C, vorzugsweise im Bereich von 65 bis 80 °C und noch besser im Bereich von 67 bis 85 °C, und
- ein drittes flüchtiges Siliconöl mit einem Flammpunkt über 80 °C, vorzugsweise mindestens 80 °C und höchstens 95 °C und besser im Bereich von 87 bis 95 °C.

31. Make-up nach dem vorhergehenden Anspruch, **dadurch gekennzeichnet, dass** das erste flüchtige Kohlenwasserstofföl das Isododecan ist.

32. Make-up nach Anspruch 30, **dadurch gekennzeichnet, dass** das zweite Siliconöl unter Decamethylcyclopentasiloxan, Decamethyltetrasiloxan und vorzugsweise Decamethylcyclopentasiloxan ausgewählt ist.

33. Make-up nach Anspruch 30, **dadurch gekennzeichnet, dass** das dritte Siliconöl das Dodecamethylcyclohexasiloxan ist.

34. Make-up nach einem der Ansprüche 30 bis 33, **dadurch gekennzeichnet, dass** das zweite flüchtige Siliconöl in einer Menge von 0,1 bis 31,9 Gew.-%, bezogen auf das Gesamtgewicht der Emulsion, vorzugsweise 5 bis 20 Gew.-% und noch bevorzugter 8 bis 16 Gew.-% vorliegt.

35. Make-up nach einem der Ansprüche 30 bis 34, **dadurch gekennzeichnet, dass** das dritte flüchtige Siliconöl in einer Menge von 0,1 bis 31,9 Gew.-%, bezogen auf das Gesamtgewicht der Emulsion, vorzugsweise 5 bis 20 Gew.-% und noch bevorzugter 8 bis 16 Gew.-% enthalten ist.

36. Make-up nach einem der vorhergehenden Ansprüche, **dadurch gekennzeichnet, dass** die flüchtige Fettphase mindestens 30 Gew.-% eines Gemisches von Decamethylcyclopentasiloxan, Dodecamethylcyclohexasiloxan und Isododecan, bezogen auf das Gesamtgewicht der Emulsion, enthält, wobei der Mengenanteil des Isododecan mindestens 6 Gew.-%, bezogen auf das Gesamtgewicht der Emulsion, beträgt.

37. Make-up nach Anspruch 36, **dadurch gekennzeichnet, dass** der Mengenanteil des Isododecan mindestens 10 Gew.-%, bezogen auf das Gesamtgewicht der Emulsion, beträgt.

38. Make-up nach einem der vorhergehenden Ansprüche, **dadurch gekennzeichnet, dass** es ein zusätzliches nicht flüchtiges Öl enthält.

39. Make-up nach Anspruch 38, **dadurch gekennzeichnet, dass** das zusätzliche Öl unter den nicht flüchtigen Kohlenwasserstoffölen, den nicht flüchtigen Siliconölen und deren Gemischen ausgewählt ist.

40. Make-up nach Anspruch 38 oder 39, **dadurch gekennzeichnet, dass** das zusätzliche Öl in einer Menge von 0,1 bis 12 Gew.-%, bezogen auf das Gesamtgewicht der Emulsion, und vorzugsweise 1 bis 5 Gew.-% enthalten ist.

41. Make-up nach einem der vorhergehenden Ansprüche, **dadurch gekennzeichnet, dass** es das Öl in einer Menge von 30 bis 45 Gew.-%, bezogen auf das Gesamtgewicht der Emulsion, und vorzugsweise 30 bis 40 Gew.-% enthält.

42. Make-up nach einem der vorhergehenden Ansprüche, **dadurch gekennzeichnet, dass** es eine Fettsubstanz enthält, die unter den Wachsen, Gummis, pastösen Fettsubstanzen und deren Gemischen ausgewählt ist.

43. Make-up nach einem der vorhergehenden Ansprüche, **dadurch gekennzeichnet, dass** es ein Verdickungsmittel für die Fettphase enthält.

44. Make-up nach Anspruch 43, **dadurch gekennzeichnet, dass** das Verdickungsmittel unter den organomodifizierten Tonen und hydrophober pyrogener Kieselsäure ausgewählt ist.

45. Make-up nach Anspruch 43 oder 44, **dadurch gekennzeichnet, dass** das Verdickungsmittel für die Fettphase in einer Menge von 0,1 bis 5 Gew.-%, bezogen auf das Gesamtgewicht der Emulsion, und besser 0,4 bis 3 Gew.-% enthalten ist.

46. Make-up nach einem der vorhergehenden Ansprüche, **dadurch gekennzeichnet, dass** die Fettphase 22 bis 50 Gew.-%, bezogen auf das Gesamtgewicht der Emulsion, vorzugsweise 25 bis 45 Gew.-% und bevorzugt 30 bis 40 Gew.-% ausmacht.

47. Make-up nach einem der vorhergehenden Ansprüche, **dadurch gekennzeichnet, dass** die wässrige Phase in einer Menge von 30 bis 50 Gew.-%, vorzugsweise 35 bis 45 Gew.-%, bezogen auf das Gesamtgewicht der Emulsion, vorliegt.

48. Make-up nach einem der vorhergehenden Ansprüche, **dadurch gekennzeichnet, dass** die wässrige Phase Wasser und/oder ein Lösungsmittel, das unter den primären Alkoholen, Glykolen, Glykolethern und deren Gemischen ausgewählt ist, und/oder ein Stabilisierungsmittel enthält.

49. Make-up nach einem der vorhergehenden Ansprüche, **dadurch gekennzeichnet, dass** es Füllstoffe enthält.

50. Make-up nach Anspruch 49, **dadurch gekennzeichnet, dass** die Füllstoffe unter Talk, Glimmer, Kieselsäure, Kaolin, Stärke, Bornitrid, Calciumcarbonat, Magnesiumcarbonat, Magnesiumhydrogencarbonat, mikrokristalliner Cellulose, Polyethylenpulvern, Polyestern, Polyamiden, Polytetrafluorethylen, Siliconpulvern und deren Gemischen ausgewählt sind.

**51.** Make-up nach Anspruch 49 oder 50, **dadurch gekennzeichnet, dass** die Füllstoffe in einer Menge von 0,1 bis 15 Gew.-%, bezogen auf das Gesamtgewicht der Emulsion, und vorzugsweise 1 bis 10 % vorliegen.

**52.** Make-up nach einem der vorhergehenden Ansprüche, **dadurch gekennzeichnet, dass** es ferner mindestens einen Zusatzstoff enthält, der unter den Gelbildnern, hydrophilen oder lipophilen Verdickungsmitteln, Hydratisierungsmitteln; Emollientien; hydrophilen oder lipophilen Wirkstoffen; Radialfängern für freie Radikale; Maskierungsmitteln; Antioxidantien; Konservierungsmitteln, Alkalisierungsmitteln oder Ansäuerungsmitteln; Parfums; Filmbildnern; löslichen Farbmitteln und deren Gemischen ausgewählt ist.

**53.** Make-up nach einem der vorhergehenden Ansprüche, **dadurch gekennzeichnet, dass** es eine bei 25 °C bei einer Schergeschwindigkeit von 200 $min^{-1}$ gemessene Viskosität von 0,15 bis 0,6 Pa·s und vorzugsweise 0,25 bis 0,45 Pa·s aufweist.

**54.** Nichttherapeutisches kosmetisches Verfahren zum Schminken der Haut, das das Auftragen eines Make-up nach einem der vorhergehenden Ansprüche auf die Haut umfasst.

**55.** Verwendung eines Make-up nach einem der Ansprüche 1 bis 53, um die Haut homogen zu schminken.

**56.** Verwendung eines $C_{8-22}$-Alkyldimethiconcopolyols und eines Dimethiconcopolyols in einem Make-up, das in Form einer Wasser-in-Öl-Emulsion vorliegt, die eine Fettphase, eine wässrige Phase und umhüllte hydrophobe Pigmente enthält, wobei die Fettphase mindestens 30 Gew.-% flüchtige Fettphase, bezogen auf das Gesamtgewicht der Emulsion, enthält, welche aufweist:

- mindestens 6 Gew.-% mindestens eines flüchtigen Kohlenwasserstofföls, bezogen auf das Gesamtgewicht der Emulsion, und
- mindestens ein flüchtiges Öl, das unter den flüchtigen Siliconölen, flüchtigen fluorierten Ölen und deren Gemischen ausgewählt ist,

wobei das Dimethiconcopolyol in einer Menge von 5 bis 10 Gew.-%, bezogen auf das Gesamtgewicht der Emulsion, enthalten ist,
um eine stabile und/oder homogene Emulsion herzustellen und/oder die Haut homogen zu schminken.

## Claims

**1.** Foundation in the form of a water-in-oil emulsion comprising a fatty phase, an aqueous phase, a $C_8$-$C_{22}$ alkyl dimethicone copolyol, a dimethicone copolyol present in an amount ranging from 5% to 10% by weight, relative to the total weight of the emulsion, hydrophobic coated pigments, the fatty phase comprising at least 30% by weight, relative to the total weight of the emulsion, of volatile fatty phase comprising:

- at least 6% by weight, relative to the total weight of the emulsion, of at least one volatile hydrocarbon oil, and
- at least one volatile oil chosen from volatile silicone oils, volatile fluorinated oils, and mixtures thereof.

**2.** Foundation according to Claim 1, **characterized in that** the $C_8$-$C_{22}$ alkyl dimethicone copolyol is a compound of the following formula (I):

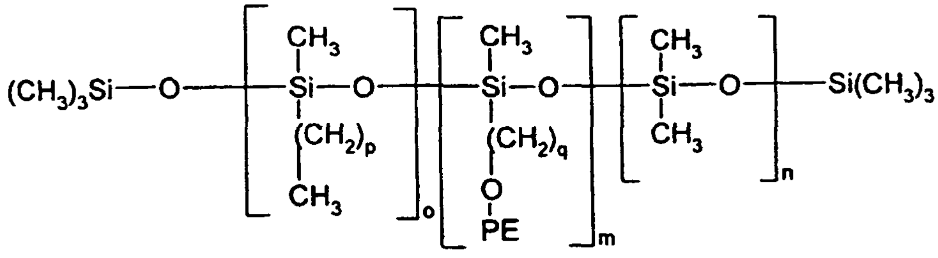

(I)

in which:

- PE represents $(-C_2H_4O)_x-C_3H_6O)_y-R$, R being chosen from a hydrogen atom and an alkyl radical of 1 to 4 carbon atoms, x ranging from 0 to 100 and y ranging from 0 to 80, x and y not being simultaneously 0
- m ranges from 1 to 40
- n ranges from 10 to 200
- o ranges from 1 to 100
- p ranges from 7 to 21
- q ranges from 0 to 4.

**3.** Foundation according to Claim 2, **characterized in that** R = H; m = 1 to 10; n = 10 to 100; o = 1 to 30; p = 15; q = 3.

**4.** Foundation according to any one of the preceding claims, **characterized in that** the $C_8$-$C_{22}$ alkyl dimethicone copolyol is cetyl dimethicone copolyol.

**5.** Foundation according to any one of the preceding claims, **characterized in that** the $C_8$-$C_{22}$ alkyl dimethicone copolyol is present in an amount ranging from 0.5% to 2% by weight, relative to the total weight of the emulsion, in particular ranging from 0.6% to 2% by weight, even better ranging from 0.7% to 2% by weight, or even ranging from 0.8% to 2% by weight, and preferably ranging from 0.5% to 1.5% by weight, in particular ranging from 0.6% to 1.5% by weight, even better ranging from 0.7% to 1.5% by weight, or even ranging from 0.8% to 1.5% by weight.

**6.** Foundation according to any one of the preceding claims, **characterized in that** the dimethicone copolyol is a compound of the following formula (II):

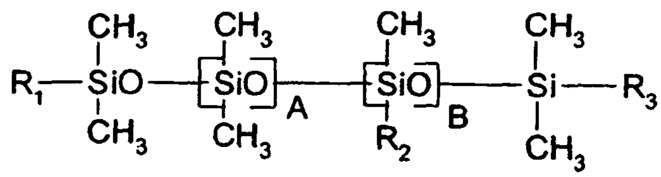

(II)

in which:

$R_1$, $R_2$, $R_3$, independently of each other, represent a $C_1$-$C_6$ alkyl radical or a radical $-(CH_2)_x-(OCH_2CH_2)_y-(OCH_2CH_2CH_2)_z-OR_4$, at least one radical $R_1$, $R_2$ or $R_3$ not being an alkyl radical; $R_4$ being hydrogen, a $C_1$-$C_3$ alkyl radical or a $C_2$-$C_4$ acyl radical;
A is an integer ranging from 0 to 200;
B is an integer ranging from 0 to 50; provided that A and B are not equal to zero at the same time;
x is an integer ranging from 1 to 6;
y is an integer ranging from 1 to 30;
z is an integer ranging from 0 to 5.

**7.** Foundation according to Claim 6, **characterized in that** $R_1 = R_3$ = methyl radical, x is an integer ranging from 2 to 6 and y is an integer ranging from 4 to 30.

**8.** Foundation according to Claim 6 or 7, **characterized in that** $R_4$ is hydrogen.

**9.** Foundation according to any one of Claims 1 to 6, **characterized in that** the dimethicone copolyol is a compound of the following formula (III):

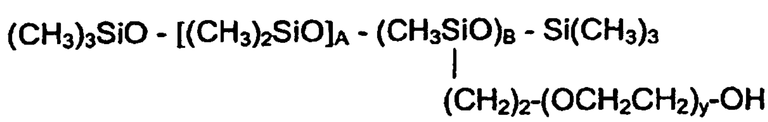

(III)

in which A is an integer ranging from 20 to 105, B is an integer ranging from 2 to 10 and y is an integer ranging

from 10 to 20.

**10.** Foundation according to any one of Claims 1 to 6, **characterized in that** the dimethicone copolyol is a compound of the following formula (IV):

$$HO\text{-} (CH_2CH_2O)_y\text{-}(CH_2)_3\text{-}[(CH_3)_2SiO]_{A'}\text{-}[(CH_3)_2Si]\text{-}(CH_2)_3\text{-}(OCH_2CH_2)_y\text{-}OH \qquad (IV)$$

in which A' and y are integers ranging from 10 to 20.

**11.** Foundation according to any one of the preceding claims, **characterized in that** the dimethicone copolyol is present in an amount ranging from 5% to 8% by weight, relative to the total weight of the emulsion, preferably ranging from 5% to 7% by weight.

**12.** Foundation according to any one of the preceding claims, **characterized in that** the hydrophobic coated pigments are chosen from metal oxides, manganese violet, ultramarine blue, Prussian blue, ultramarine blue, ferric blue, bismuth oxychloride, pearl, mica coated with titanium or with bismuth oxychloride, coloured pearlescent pigments, and mixtures thereof.

**13.** Foundation according to any one of the preceding claims, **characterized in that** the hydrophobic coated pigments are chosen from iron oxides and titanium dioxides.

**14.** Foundation according to any one of the preceding claims, **characterized in that** the hydrophobic coated pigments are treated with a hydrophobic agent chosen from silicones, fatty acids, metal soaps, perfluoroalkyl phosphates, perfluoroalkylsilanes, perfluoroalkylsilazanes, polyhexafluoropropylene oxides, polyorganosiloxanes comprising perfluoroalkyl perfluoropolyether groups, amino acids; N-acylated amino acids or their salts; lecithin, isopropyl triisostearyl titanate, and mixtures thereof.

**15.** Foundation according to Claim 14, **characterized in that** the N-acylated amino acids comprise an acyl group having from 8 to 22 carbon atoms.

**16.** Foundation according to any one of the preceding claims, **characterized in that** the hydrophobic coated pigments are present in an amount ranging from 0.5% to 20% by weight, relative to the total weight of the emulsion, preferably in an amount at least equal to 5% by weight, and preferentially ranging from 5% to 20% by weight.

**17.** Foundation according to any one of the preceding claims, **characterized in that** the volatile fatty phase is present in an amount ranging from 30% to 45% by weight, relative to the total weight of the emulsion, preferably ranging from 30% to 40% by weight and preferentially ranging from 33% to 38% by weight.

**18.** Foundation according to any one of the preceding claims, **characterized in that** the volatile hydrocarbon oil is chosen from hydrocarbon oils having a flash point ranging from 40°C to 102°C, preferably ranging from 40°C to 55°C, and preferentially ranging from 40°C to 50°C.

**19.** Foundation according to any one of the preceding claims, **characterized in that** the volatile hydrocarbon oil is chosen from volatile hydrocarbon oils having from 8 to 16 carbon atoms, and mixtures thereof.

**20.** Foundation according to any one of the preceding claims, **characterized in that** the volatile hydrocarbon oil is chosen from branched $C_8$-$C_{16}$ alkanes, branched $C_8$-$C_{16}$ esters, and mixtures thereof.

**21.** Foundation according to any one of the preceding claims, **characterized in that** the volatile hydrocarbon oil is chosen from isododecane, isodecane and isohexadecane.

**22.** Foundation according to any one of the preceding claims, **characterized in that** the volatile hydrocarbon oil is isododecane.

**23.** Foundation according to any one of the preceding claims, **characterized in that** the volatile hydrocarbon oil is present in an amount ranging from 6% to 25% by weight, relative to the total weight of the emulsion, preferably ranging from 10% to 20% by weight, and preferentially ranging from 10% to 15% by weight.

**24.** Foundation according to any one of the preceding claims, **characterized in that** the volatile silicone oil is chosen from silicone oils having a flash point ranging from 40°C to 102°C, preferably having a flash point greater than 55°C and less than or equal to 95°C, and preferentially ranging from 65°C to 95°C.

**25.** Foundation according to any one of the preceding claims, **characterized in that** the volatile silicone oil is chosen from linear or cyclic silicone oils having from 2 to 7 silicon atoms, these silicones optionally containing alkyl or alkoxy groups having from 1 to 10 carbon atoms.

**26.** Foundation according to any one of the preceding claims, **characterized in that** the volatile silicone oil is chosen from octamethylcyclotetrasiloxane, decamethylcyclopentasiloxane, dodecamethylcyclohexasiloxane, heptamethylhexyltrisiloxane, hepta-methyloctyltrisiloxane, hexamethyldisiloxane, octamethyltrisiloxane, decamethyltetrasiloxane, dodeca-methylpentasiloxane, and mixtures thereof.

**27.** Foundation according to any one of the preceding claims, **characterized in that** the volatile fluorinated oil is chosen from nonafluoroethoxybutane, nonafluoromethoxybutane, decafluoropentane, tetradecafluorohexane, dodecafluoropentane, and mixtures thereof.

**28.** Foundation according to any one of the preceding claims, **characterized in that** the volatile fatty phase comprises at least one volatile hydrocarbon oil and at least one volatile silicone oil.

**29.** Foundation according to any one of the preceding claims, **characterized in that** the volatile oil chosen from volatile silicone oils, volatile fluorinated oils, and mixtures thereof is present in an amount ranging from 20% to 32% by weight, relative to the total weight of the emulsion, preferably ranging from 20% to 30% by weight, and preferentially ranging from 22% to 26% by weight.

**30.** Foundation according to the preceding claim, **characterized in that** the volatile fatty phase comprises:

- a first volatile hydrocarbon oil,
- a second volatile silicone oil having a flash point greater than 55°C and less than or equal to 80°C, preferably ranging from 65°C to 80°C, and even better ranging from 67°C to 85°C,
- a third volatile silicone oil having a flash point greater than 80°C, preferably greater than or equal to 80°C and less than or equal to 95°C, and even better ranging from 87°C to 95°C.

**31.** Foundation according to the preceding claim, **characterized in that** the first volatile hydrocarbon oil is isododecane.

**32.** Foundation according to Claim 30, **characterized in that** the second silicone oil is chosen from decamethylcyclopentasiloxane, decamethyltetrasiloxane, and preferably decamethylcyclopentasiloxane.

**33.** Foundation according to Claim 30, **characterized in that** the third silicone oil is dodecamethylcyclohexasiloxane.

**34.** Foundation according to any one of Claims 30 to 33, **characterized in that** the second volatile silicone oil is present in an amount ranging from 0.1% to 31.9% by weight, relative to the total weight of the emulsion, preferably ranging from 5% to 20% by weight, and preferentially ranging from 8% to 16% by weight.

**35.** Foundation according to any one of Claims 30 to 34, **characterized in that** the third volatile silicone oil is present in an amount ranging from 0.1% to 31.9% by weight, relative to the total weight of the emulsion, preferably ranging from 5% to 20% by weight, and preferentially ranging from 8% to 16% by weight.

**36.** Foundation according to any one of the preceding claims, **characterized in that** the volatile fatty phase comprises at least 30% by weight, relative to the total weight of the emulsion, of a mixture of decamethylcyclopentasiloxane, dodecamethylcyclohexasiloxane and isododecane, the content of isododecane being at least 6% by weight, relative to the total weight of the emulsion.

**37.** Foundation according to Claim 36, **characterized in that** the isododecane content is at least 10% by weight, relative to the total weight of the emulsion.

**38.** Foundation according to any one of the preceding claims, **characterized in that** it comprises a non-volatile additional oil.

**39.** Foundation according to Claim 38, **characterized in that** the additional oil is chosen from non-volatile hydrocarbon oils, non-volatile silicone oils, and mixtures thereof.

**40.** Foundation according to Claim 38 or 39,
**characterized in that** the additional oil is present in an amount ranging from 0.1% to 12% by weight, relative to the total weight of the emulsion, and preferably ranging from 1% to 5% by weight.

**41.** Foundation according to any one of the preceding claims, **characterized in that** it comprises oils in an amount ranging from 30% to 45% by weight, relative to the total weight of the emulsion, and preferably ranging from 30% to 40% by weight.

**42.** Foundation according to any one of the preceding claims, **characterized in that** it comprises a fatty substance chosen from waxes, gums, pasty fatty substances, and mixtures thereof.

**43.** Foundation according to any one of the preceding claims, **characterized in that** it comprises a fatty phase thickening agent.

**44.** Foundation according to Claim 43, **characterized in that** the thickening agent is chosen from organomodified clays and hydrophobic pyrogenic silica.

**45.** Foundation according to Claim 43 or 44, **characterized in that** the fatty phase thickening agent is present in an amount ranging from 0.1% to 5% by weight relative to the total weight of the emulsion, and even better from 0.4% to 3% by weight.

**46.** Foundation according to any one of the preceding claims, **characterized in that** the fatty phase represents from 22% to 50% by weight, relative to the total weight of the emulsion, preferably from 25% to 45% by weight, and preferentially from 30% to 40% by weight.

**47.** Foundation according to any one of the preceding claims, **characterized in that** the aqueous phase is present in an amount ranging from 30% to 50% by weight, preferably ranging from 35% to 45% by weight, relative to the total weight of the emulsion.

**48.** Foundation according to any one of the preceding claims, **characterized in that** the aqueous phase comprises water and/or a solvent chosen from primary alcohols, glycols, glycol ethers, and mixtures thereof, and/or a stabilizing agent.

**49.** Foundation according to any one of the preceding claims, **characterized in that** it comprises fillers.

**50.** Foundation according to Claim 49, **characterized in that** the fillers are chosen from talc, mica, silica, kaolin, starch, boron nitride, calcium carbonate, magnesium carbonate or hydrocarbonate, microcrystalline cellulose, polyethylene powders, polyesters, polyamides, polytetrafluoroethylene, silicone powders, and mixtures thereof.

**51.** Foundation according to Claim 49 or 50,
**characterized in that** the fillers are present in an amount ranging from 0.1% to 15% by weight, relative to the total weight of the emulsion, preferably 0.1% to 10%.

**52.** Foundation according to any one of the preceding claims, **characterized in that** it comprises in addition at least one additive chosen from gelling agents, hydrophilic or lipophilic thickening agents, moisturizing agents; emollients; hydrophilic or lipophilic active agents; anti-free radical agents; sequestrants; antioxidants; preservatives; basifying or acidifying agents; perfumes; film-forming agents; soluble colorants, and mixtures thereof.

**53.** Foundation according to any one of the preceding claims, **characterized in that** it has a viscosity, measured at

25°C, at a shear rate of 200 $min^{-1}$, ranging from 0.15 to 0.6 Pa.s, and preferably ranging from 0.25 to 0.45 Pa.s.

**54.** Cosmetic method for the non-therapeutic application of make-up to the skin which comprises the application to the skin of a foundation according to any one of the preceding claims.

**55.** Use of a foundation according to any one of Claims 1 to 53 in order to obtain a homogeneous make-up on the skin.

**56.** Use of a $C_8$-$C_{22}$ alkyl dimethicone copolyol and of a dimethicone copolyol in a foundation composition in the form of a water-in-oil emulsion containing a fatty phase, an aqueous phase, hydrophobic coated pigments, the fatty phase comprising at least 30% by weight, relative to the total weight of the emulsion, of volatile fatty phase comprising:

- at least 6% by weight, relative to the total weight of the emulsion, of at least one volatile hydrocarbon oil, and
- at least one volatile oil chosen from volatile silicone oils, volatile fluorinated oils, and mixtures thereof,
  the dimethicone copolyol being present in an amount ranging from 5% to 10% by weight, relative to the total weight of the emulsion,
  in order to obtain a stable and/or homogeneous emulsion and/or in order to obtain a homogeneous make-up on the skin.